# EUROPEAN PATENT APPLICATION

(11) **EP 3 144 279 A1**
(43) Date of publication of application: **22.03.2017**
(21) Application number: 15780571.4
(22) Date of filing: 18.03.2015
(51) Int. Cl.: C01G 39/06, C01B 21/064, C01B 33/40, C01B 33/42, C01G 17/00, C01G 19/00, C01G 25/00, C01G 33/00, C01G 39/00, C07D 233/58, C07D 233/60

(54) **LAYERED SUBSTANCE-CONTAINING LIQUID AND METHOD FOR PRODUCING SAME**

(30) Priority: 17.04.2014 JP 2014085213
(71) Applicant: Adeka Corporation, Tokyo 116-8554 (JP)
(72) Inventor: KOBAYASHI, Atsushi, Tokyo 116-8554 (JP); TANIUCHI, Ryo, Tokyo 116-8554 (JP); AOYAMA, Yohei, Tokyo 116-8554 (JP)
(74) Representative: Müller Hoffmann & Partner
(86) International application number: PCT/JP2015/058011
(87) International publication number: WO 2015/159635

(57) **Abstract**

A laminate of layered substances each containing two or more kinds of elements as constituent elements is contained in an ionic liquid containing a specific cation, and the ionic liquid containing the laminate is irradiated with one or both of sonic waves and electric waves.

## Description

### Technical Field

The present invention relates to a layered substance-containing liquid containing an ionic liquid together with a layered substance, and a method for producing the same.

### Background Art

A substance having a layered structure (layered substance) exhibits characteristic physical properties resulting from the layered structure, and many researchers have been conducting research on various layered substances.

In particular, recently, there has been proposed to use a layered substance called "nanosheet" for improvement of performance of electronic devices (for example, refer to Non-Patent Literature 1). A laminate of a plurality of (two to five) layers of layered substances, as well as a single-layer (one layer) layered substance, is used as the nanosheet.

Accordingly, attention has been focused on layered substances having various kinds of compositions, specifically, a layered substance containing one kind of element as a constituent element (a single-element layered substance) and a layered substance containing two or more kinds of elements as constituent elements (a multiple-element layered substance). Examples of the single-element layered substance may include graphene. Examples of the multiple-element layered substance may include a chalcogenide-based layered substance typified by molybdenite.

These layered substances are generally present in a state in which a plurality of layered substances are laminated (a laminate). Accordingly, in order to peel the layered substance from the laminate, there have been proposed a physical peeling method using an adhesive tape, a chemical peeling method using an oxidation method, a method of applying ultrasonic waves or any other waves in an organic solvent, and any other method (for example, refer to Patent Literature 1).

### Citation list

### Non-Patent Literature

[Non-Patent Literature 1] B.Radisavljevic et al., Nature Nanotech, 6, pp. 147 to 150, 2011

### Patent Literature

Patent Literature 1: International Publication No. WO 2013/172350

### Summary

Although a technology to obtain the single-element layered substance in the layered substances is well known, a technology to obtain the multiple-element layered substance is not well known, and a technology that makes it possible to obtain the multiple-element layered substance is desired.

An object of the present invention is to provide a layered substance-containing liquid that makes it possible to easily obtain a layered substance containing two or more kinds of elements as constituent elements, and a method for producing the same.

As a result of intensive studies to achieve the foregoing object, the inventors of the present invention have found out that the foregoing issue is solved by using, as a dispersion medium, an ionic liquid containing a specific compound and irradiating the ionic liquid with sonic waves or/and any other waves.

The present invention is based on the foregoing findings, and the layered substance-containing liquid of the present invention includes: an ionic liquid containing a cation represented by the following formula (1) and a layered substance containing two or more kinds of elements as constituent elements. 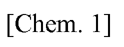 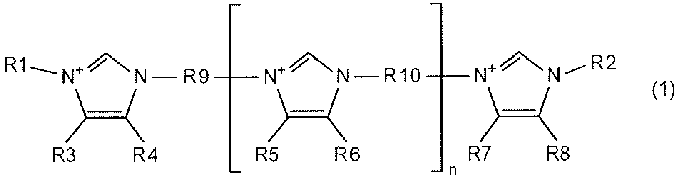 where each of R1 and R2 is a monovalent hydrocarbon group, each of R3 to R8 is one of a hydrogen atom and a monovalent hydrocarbon group, each of R9 and R10 is one of a divalent group represented by the following formula (2) and a divalent group represented by the following formula (3), and n is an integer of 0 or more. 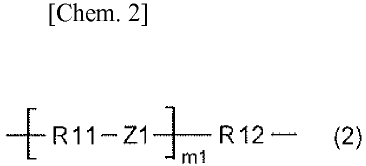 where each of R11 and R12 is a divalent hydrocarbon group, Z1 is one of an ether bond, a thioether bond, a divalent aromatic hydrocarbon group, and m1 is an integer of 0 or more. 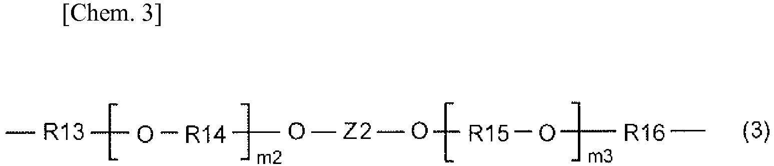 where each of R13 to R16 is a divalent hydrocarbon group, Z2 is a divalent aromatic hydrocarbon group, each of m2 and m3 is an integer of 1 or more.

Moreover, a method for producing a layered substance-containing liquid of the present invention includes: containing a laminate of layered substances in an ionic liquid containing a cation represented by the foregoing formula (1), the layered substances each containing two or more kinds of elements as constituent elements; and irradiating the ionic liquid containing the laminate with one or both of sonic waves and electric waves.

Herein, the "layered substance" of the present invention is a layered (thin) substance (multiple-element layered substance) containing two or more kinds of elements as constituent elements, and may be a single-layer layered substance or a multilayer layered substance. However, each of layers of the multilayer layered substance contains two or more kinds of elements as constituent elements. Moreover, the number of layers of the multilayer layered substance is sufficiently small, that is, nine or less, and may be preferably four or less. In contrast, the "laminate of layered substances" of the present invention is a laminate of a plurality of layered substances; therefore, the laminate includes multiple layers.

According to the layered substance-containing liquid and the method for producing the same of the present invention, the ionic liquid containing a specific cation contains the laminate of the layered substances, and the ionic liquid containing the laminate is irradiated with sonic waves or/and any other waves, which causes the layered substances to be dispersed in the ionic liquid with a high concentration. This makes it possible to easily obtain the layered substances even in a case in which the layered substances each contain two or more kinds of elements as constituent elements.

### Brief Description of Drawings

[FIG. 1] FIG. 1 is an XRD chart (before irradiation with microwaves) of Experimental Example 1-1 (MoS₂).
[FIG. 2] FIG. 2 is an XRD chart (after irradiation with microwaves) of Experimental Example 1-1 (MoS₂).
[FIG. 3] FIG. 3 is an XRD chart (before irradiation with microwaves) of Experimental Example 1-2 (BN).
[FIG. 4] FIG. 4 is an XRD chart (after irradiation with microwaves) of Experimental Example 1-2 (BN).

### Modes for Carrying out the Invention

In the following, some embodiments of the present invention are described in detail in the following order. Specific description of the present invention is not limited to embodiments to be described below, and the present invention may be modified as appropriate.

1. Layered Substance-containing Liquid
   1-1. Ionic Liquid
      1-1-1. Cation
      1-1-2. Anion
   1-2. Layered Substance
   1-3. Other Materials
2. Method for Producing Layered Substance-containing Liquid
   2-1. Preparation of Layered Substance-containing Liquid
   2-2. Purification of Layered Substance-containing Liquid
3. Action and Effects

### <1. Layered Substance-containing Liquid>

First, description is given of a configuration of a layered substance-containing liquid.

The layered substance-containing liquid includes an ionic liquid and a layered substance, and the layered substance is dispersed in the ionic liquid.

### <1-1. Ionic Liquid>

The ionic liquid is a liquid salt, and includes a cation and an anion.

### <1-1-1. Cation>

The cation includes one or more kinds of positive ions represented by the following formula (1). 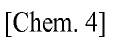 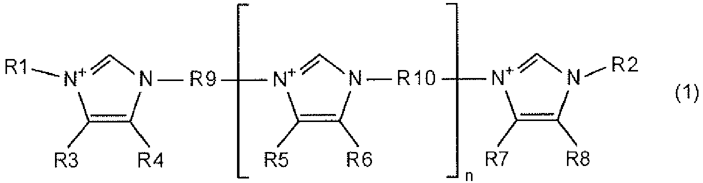 where each of R1 and R2 is a monovalent hydrocarbon group, each of R3 to R8 is one of a hydrogen atom and a monovalent hydrocarbon group, each of R9 and R10 is one of a divalent group represented by the following formula (2) and a divalent group represented by the following formula (3), and n is an integer of 0 or more. 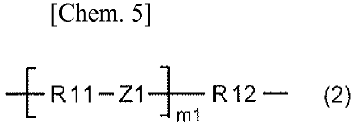 where each of R11 and R12 is a divalent hydrocarbon group, Z1 is one of an ether bond, a thioether bond, a divalent aromatic hydrocarbon group, and m1 is an integer of 0 or more. 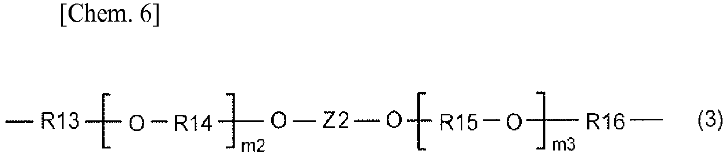 where each of R13 to R16 is a divalent hydrocarbon group, Z2 is a divalent aromatic hydrocarbon group, each of m2 and m3 is an integer of 1 or more.

The kind of each of R1 and R2 is not particularly limited as long as each of R1 and R2 is one of monovalent hydrocarbon groups. The monovalent hydrocarbon group may be a straight-chain group or a branched group having one or more side chains. R1 and R2 may be groups of a same kind or groups of different kinds.

The monovalent hydrocarbon group is a generic name for a monovalent group including carbon (C) and hydrogen (H). Examples of the monovalent hydrocarbon group may include an alkyl group, an alkenyl group, an alkynyl group, an aryl group, a cycloalkyl group, and a monovalent group in which two or more kinds of these groups are bound.

Specific examples of the alkyl group may include a methyl group, an ethyl group, a propyl group, an isopropyl group, a butyl group, an isobutyl group, an s-butyl group, a t-butyl group, an amyl group, an isoamyl group, a t-amyl group, a hexyl group, and a heptyl group. Specific examples of the alkenyl group may include a vinyl group and an allyl group. Specific examples of the alkynyl group may include an ethynyl group. Specific examples of the aryl group may include a phenyl group and a naphthyl group. Specific examples of the cycloalkyl group may include a cyclopropyl group, a cyclobutyl group, a cyclopentyl group, a cyclohexyl group, a cycloheptyl group, and a cyclooctyl group. Specific examples of the monovalent group in which two or more kinds of the groups are bound may include a group represented by the following formula (4). The group represented by the formula (4) is a monovalent group (a benzyl group) in which an aryl group (a phenyl group) and an alkyl group (a methyl group) are bound.

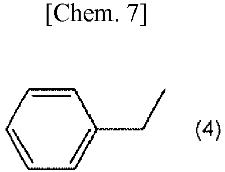

The number of carbons in the monovalent hydrocarbon group is not particularly limited, but it may be preferable that the number of carbons not be extremely too large. More specifically, the number of carbons in each of the alkyl group, the alkenyl group, and the alkynyl group may be preferably 1 to 7 both inclusive. The number of carbons in each of the aryl group and the cycloalkyl group may be preferably 6 or 7. This makes it possible to improve dispersibility and other properties of the layered substance.

The kind of each of R3 to R8 is not particularly limited as long as each of R3 to R8 is one of a hydrogen atom and a monovalent hydrocarbon group. R3 to R8 may be groups of a same kind or groups of different kinds. Some of R3 to R8 may be groups of a same kind. Details of the monovalent hydrocarbon group are as described above.

The value of n that determines the number of repeating units is not particularly limited as long as the value of n is an integer of 0 or more. In particular, n may be preferably an integer of 30 or less. This makes it possible to improve dispersibility and other properties of the layered substance.

Each of R9 and R10 may be the divalent group represented by the formula (2) or the divalent group represented by the formula (3). R9 and R10 may be groups of a same kind or groups of different kinds. Accordingly, in a case in which n is 2 or more and a plurality of R10's are therefore present, the plurality of R10's may be groups of a same kind or a groups of different kinds, or some of the plurality of R10's may be groups of a same kind.

The kind of each of R11 and R12 is not particularly limited as long as each of R11 and R12 is one of divalent hydrocarbon groups. The divalent hydrocarbon group may be a straight-chain group or a branched group having one or more side chains. R11 and R12 may be groups of a same kind or groups of different kinds. Accordingly, in a case in which m1 is 2 or more and a plurality of R11's are therefore present, the plurality of R11's may be groups of a same kind or groups of different kinds, or some of the plurality of R11's may be groups of a same kind.

The divalent hydrocarbon group is a generic name for a divalent group including carbon and hydrogen. Examples of the divalent hydrocarbon group may include an alkylene group, an alkenylene group, an alkynylene group, an arylene group, a cycloalkylene group, and a divalent group in which two or more kinds of these groups are bound.

Specific examples of the alkylene group may include a methane-1,1-diyl group, an ethane-1,2-diyl group, a propane-1,3-diyl group, a butane-1,4-diyl group, an ethane-1,1-diyl group, a propane-1,2-diyl group, a butane-1,2-diyl group, a butane-1,3-diyl group, and butane-2,3-diyl group. Specific examples of the alkenylene group may include a vinylene group. Specific examples of the alkynylene group may include an ethynylene group. Specific examples of the arylene group may include a phenylene group and a naphthylene group. Specific examples of the cycloalkylene group may include a cyclopropylene group and a cyclobutylene group. Specific examples of the divalent group in which two or more kinds of the groups are bound may include a group represented by the following formula (5). The group represented by the formula (5) is a divalent group in which one arylene group (a phenylene group) and two alkylene groups (ethane-1,2-diyl groups) are bound.

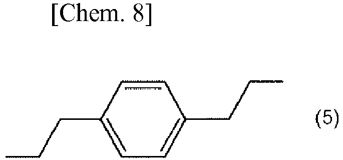

The number of carbons in the divalent hydrocarbon group is not particularly limited, but it may be preferable that the number of carbons not be extremely too large. More specifically, the number of carbons in each of the alkylene group, the alkenylene group, and the alkynylene group may be preferably 1 to 4 both inclusive. The number of carbons in each of the arylene group and the cycloalkylene group may be preferably 6. This makes it possible to improve dispersibility and other properties of the layered substance.

The kind of Z1 is not particularly limited as long as Z1 is one of the ether bond, the thioether bond, and the divalent aromatic hydrocarbon group. Accordingly, in a case in which m1 is 2 or more and a plurality of Z1's are therefore present, the plurality of Z1's may be groups of a same kind or groups of different kinds, or some of the plurality of Z1's may be groups of a same kind.

The divalent aromatic hydrocarbon group is a generic name for a divalent group including carbon and hydrogen and having a cyclic conjugated structure. Examples of the divalent aromatic hydrocarbon group may include an arylene group and a divalent group in which two or more kinds of arylene groups are bound. Specific examples of the arylene group may include a phenylene ring as a monocyclic arylene group and a naphthylene group as a polycyclic arylene group.

The divalent aromatic hydrocarbon group has two atomic bondings, but the positions of the two atomic bondings are not particularly limited. To give an example, in a case in which the divalent aromatic hydrocarbon group is a phenylene group, a position of a first atomic bonding of the atomic bondings with respect to a position of a second atomic bonding may be in an ortho-position, a meta-position, or a para-position. In particular, the position of the first atomic bonding may be preferably in a para-position. This makes it possible to improve chemical stability of the ionic liquid, and dispersibility and other properties.

The value of m1 that determines the number of repeating units is not particularly limited as long as m1 is an integer of 0 or more. In particular, m1 may be preferably an integer of 30 or less. This makes it possible to improve dispersibility and other properties of the layered substance.

The kind of each of R13 to R16 is not particularly limited as long as each of R13 to R16 is one of divalent hydrocarbon groups. R13 to R16 may be groups of a same kind or groups of different kinds, or some of R13 to R16 may be groups of a same kind. Accordingly, in a case in which m2 is 2 or more and a plurality of R14's are therefore present, the plurality of R14's may be groups of a same kind or groups of different kinds, or some of the plurality of R14's may be groups of a same kind. Likewise, in a case in which m3 is 2 or more and a plurality of R15's are therefore present, the plurality of R15's may be groups of a same kind or groups of different kinds, or some of the plurality of R15's may be groups of a same kind. Details of the divalent hydrocarbon group are as described above.

The kind of Z2 is not particularly limited as long as Z2 is one of divalent aromatic hydrocarbon groups. Details of the divalent aromatic hydrocarbon group are as described above.

The value of each of m2 and m3 that determines the number of repeating units is not particularly limited as long as each of m2 and m3 is an integer of 1 or more. In particular, each of m2 and m3 may be preferably an integer of 30 or less. This makes it possible to improve dispersibility and other properties of the layered substance.

In particular, the composition of the cation may preferably satisfy the following condition, which makes it possible to easily achieve synthesis of the ionic liquid and to further improve dispersibility and other properties of the layered substance.

Each of R1 and R2 positioned at both ends may be preferably a straight-chain alkyl group, and more specifically, each of R1 and R2 may be preferably a methyl group, an ethyl group, an n-propyl group, an n-butyl group, an n-pentyl group, and an n-hexyl group. Each of R3 to R8 introduced into each imidazolium ring may be preferably a hydrogen atom. Each of R11 to R16 introduced into a group connecting adjacent imidazolium rings may be preferably a straight-chain alkylene group, and more specifically, each of R11 to R16 may be preferably an ethylene group.

The value of n that determines the number of repeating units may be preferably an integer of 0 to 2. In other words, a repeating part of the imidazolium ring of the cation may be preferably one of a dimer, a trimer, and a tetramer. A too large value of n causes an increase in viscosity of the ionic liquid, which raises a possibility that an effect by irradiation with ultrasonic waves or any other waves (easiness of peeling of the layered substance) is less likely to be achieved in a process of producing a layered substance-containing liquid to be described later. Moreover, in a case in which it is necessary to perform purification of the layered substance-containing liquid, this may cause difficulty in performing the purification.

The value of m1 may be preferably an integer of 0 to 5, and the value of each of m2 and m3 may be preferably 2 or 3.

### <1-1-2. Anion>

The anion includes one or more kinds of negative ions.

The negative ion may be represented by pAn^{q-}, for example, where An^{q-} is a q-valent negative ion, p is a coefficient necessary to maintain neutrality of the entirety of the ionic liquid, and the value of p is determined depending on the kind of the negative ion. A product (p x q) of p and q is equal to a valence of the entire cation.

Examples of monovalent negative ions may include a halogen ion, an inorganic ion, an organic sulfonic acid-based ion, and an organic phosphoric acid-based ion.

Specific examples of the halogen ion may include a chlorine ion (Cl⁻), a bromine ion (Br⁻), an iodine ion (I⁻), and a fluorine ion (F⁻).

Specific examples of the inorganic ion may include a perchlorate ion (ClO₄⁻), a chlorate ion (ClO₃⁻), a thiocyanate ion (SCN⁻), a hexafluorophosphate ion (PF₆⁻), an antimony hexafluoride ion (SbF₆⁻), and a boron tetrafluoride ion (BF4⁻).

Specific examples of the organic sulfonic acid-based ion may include a benzenesulfonate ion, a toluenesulfonate ion, a trifluoromethanesulfonate ion, a diphenylamine-4-sulfonate ion, a 2-amino-4-methyl-5-chlorobenzenesulfonate ion, and a 2-amino-5-nitrobenzenesulfonate ion. In addition, the organic sulfonic acid-based ion may be any of organic sulfonic acid-based ions described in Japanese Unexamined Patent Application Publication No. H8-253705, Japanese Unexamined Patent Application Publication (Published Japanese Translation of PCT Application) No. 2004-503379, Japanese Unexamined Patent Application Publication No. 2005-336150, International Publication No. WO 2006/28006, and other literatures.

Specific examples of the organic phosphoric acid-based ion may include an octyl phosphate ion, a dodecyl phosphate ion, an octadecyl phosphate ion, a phenyl phosphate ion, a nonylphenyl phosphate ion, and a 2,2'-methylenebis(4,6-di-tert-butylphenyl)phosphonate ion.

In addition, specific examples of the monovalent negative ions may include a bis(trifluoromethanesulfonyl) imide ion ((CF₃SO₂)₂N⁻), a bis(perfluorobutanesulfonyl) imide ion ((C₄F₉SO₂)₂N⁻), a perfluoro-4-ethylcyclohexanesulphonate ion, a tetrakis(pentafluorophenyl) borate ion, a tris(fluoroalkylsulfonyl) carboxylate ion, and a dibenzoyl tartrate anion.

Specific examples of the divalent negative ion may include a benzenedisulfonate ion and a naphthalenedisulfonate ion.

In particular, the anion may be preferably one of a chlorine ion, a bromine ion, a hexafluorophosphate ion, a boron tetrafluoride ion, and a bis(trifluoromethanesulfonyl) imide ion, and may be more preferably a hexafluorophosphate ion. This makes it possible to improve dispersibility and other properties of the layered substance.

### <1-2. Layered Substance>

The layered substance is a layered thin substance (multiple-element layered substance) containing two or more kinds of elements as constituent elements, as described above, and is a so-called nanosheet.

The layered substance is not limited to a single-layer layered substance, and may be a multilayer layered substance as long as each of layers of the multilayer layered substance contains two or more kinds of elements as constituent elements and the number of layers of the multilayer layered substance is sufficiently small. Note that the layered substance is one or more layered substances peeled, in a process of producing the layered substance-containing liquid to be described later, from a laminate having a multilayer structure in which a plurality of layered substances are laminated.

The layered substance described here (each layer in a case with the multilayer layered substance) is a multiple-element layered substance; therefore, a substance (a single-element layered substance) containing only one element as a constituent element does not correspond to the layered substance of the present invention. Examples of the single-element layered substance may include graphite.

The layered substance contains one or more kinds of the multiple-element layered substances mentioned above. Specific examples of the multiple-element layered substances may include metal chalcogenide, metal oxide-metal oxyhalide, metal phosphate, clay mineral-silicate, double hydroxide, layered titanium oxide, layered perovskite oxide, and boron nitrides.

Specific examples of the metal chalcogenide may include MX (where M is Ga, Ge, In, and other elements, and X is S, Se, Te, and other elements), MX₂ (where M is Ti, Zr, Hf, V, Nb, Ta, Mo, W, Sn, and other elements, and X is S, Se, Te, and other elements), and MPX₃ (where M is Mg, V, Mn, Fe, Co, Ni, Zn, Cd, In, and other elements, and X is S, Se, Te, and other elements).

Specific examples of the metal oxide-metal oxyhalide may include MₓO_{y} (where M is Ti, Mn, Mo, V, and other elements), MOXO₄ (where M is Ti, V, Cr, Fe, and other elements, and X is P, As, and other elements), MOX (where M is Ti, V, Cr, Fe, and other elements, and X is Cl, Br, and other elements), LnOCl (where Ln is Yb, Er, Tm, and other elements), niobate represented by K[Ca₂Naₙ₋₃NbₙO₃ₙ₊₁] (where n satisfies 3≤n<7), and titanate. Note that specific examples of MₓO_{y} may include MoO₃, Mo₁₈O₅₂, V₂O₅, LiNbO₂, and LiₓV₃O₈. Specific examples of the titanate may include K₂Ti₄O₉ and KTiNbO₅.

Specific examples of the metal phosphate may include M(HPO₄)₂ (where M is Ti, Zr, Ce, Sn, and other elements) and Zr(ROPO₃)₂ (where R is H, Rh, CH₃, and other elements).

Specific examples of the clay mineral-silicate may include a smectite group, a kaolin group, pyrophyllite-talc, vermiculite, a mica group, a brittle mica group, a chlorite group, sepiolite-palygorskite, imogolite, allophone, hisingerite, magadiite, and kanemite. Note that specific examples of the smectite group may include montmorillonite and saponite. Specific examples of the kaolin group may include kaolinite.

Specific examples of the double hydroxide may include [M²⁺₁₋ₓM³⁺ₓ(OH)₂] [An⁻]_{x/n}·zH₂O (where examples of M²⁺ may include Mg²⁺ and Zn²⁺, and examples of M3⁺ may include Al³⁺ and Fe³⁺, and An⁻ is any anion).

Specific examples of the layered titanium oxide may include potassium dititanate (K₂Ti₂O₅) and potassium tetratitanate (K₂Ti₄O₉).

Specific examples of the layered perovskite oxide may include KCa₂Nb₃O₁₀, KSr₂Nb₃O₁₀, and KLaNb₂O₇.

Boron nitrides are a generic name for a compound containing nitrogen (N) and boron (B). Specific examples of the boron nitrides may include boron nitride (BN) and boron carbon nitride (BCN).

Note that an average particle diameter of the layered substance is not particularly limited, but the average particle diameter of the layered substance may be preferably 100 µm or less, and more preferably 1 µm to 100 µm both inclusive. This makes it possible to improve dispersibility and other properties. The average particle diameter is a so-called median diameter (D50 corresponding to 50% in a cumulative particle diameter distribution).

### <1-3. Other Materials>

Note that the layered substance-containing liquid may contain one or more kinds of other materials together with the ionic liquid and the layered substance mentioned above.

The other materials may include, for example, other ionic liquids (excluding the ionic liquid mentioned above). Specific examples of the other ionic liquids may include butyl methylimidazolium hexafluorophosphate and buthyl methylimidazolium bis(trifluoromethanesulfonyl) imide.

Moreover, the other materials may include, for example, an organic solvent (excluding the ionic liquid). Specific examples of the organic solvent may include water and ethanol.

### <2. Method for Producing Layered Substance-containing Liquid>

Next, description is given of a method for producing the foregoing layered substance-containing liquid. Hereinafter, a laminate having a multilayer structure in which a plurality of layered substances are laminated is referred to as "layered laminate".

### <2-1. Preparation of Layered Substance-containing Liquid>

The layered substance-containing liquid is prepared as follows. First, an ionic liquid containing one or more kinds of the cations represented by the formula (1) may be synthesized by any synthesizing method, for example. Note that the anion may be of any kind.

To synthesize an ionic liquid in which the value of n in the formula (1) is 0, for example, triethylene glycol bis(p-toluenesulfonate ester) or any other similar material, and 1-butylimidazole or any other similar material are used as raw materials. Thus, a cation that is a dimer is obtained.

To synthesize an ionic liquid in which the value of n is 1 or more is synthesized, for example, triethylene glycol bis(p-toluenesulfonate) or any other similar material, (1,1'-[1,2-ethanediylbis(oxy-2-ethanediyl)bis(imidazole)]) or any other similar material, and 1-butylimidazole or any other similar material are used as raw materials. Thus, a cation that is a trimer, a tetramer, or any other oligomer is obtained.

It is to be noted that a method of synthesizing the ionic liquid may be described in detail in International Publication No. WO 2013/172350 and other literatures.

Next, the layered laminate is added to the ionic liquid to be contained in the ionic liquid. In this case, the ionic liquid may be stirred on as-necessary basis. Thus, the layered laminate is dispersed in the ionic liquid.

Finally, the ionic liquid containing the layered laminate is irradiated with one or both of sonic waves or electric waves.

The kind of the sonic waves is not particularly limited, but ultrasonic waves may be preferably used. This makes it possible to easily peel the layered substance from the layered laminate. In a case in which the ultrasonic waves are used, for example, any ultrasonic disperser may be used, but a horn-type ultrasonic disperser may be preferably used. Conditions of the ultrasonic waves such as frequency, amplitude, and irradiation time are not particularly limited. To give an example, the frequency of the ultrasonic wave is from 10 kHz to 1 MHz both inclusive, the amplitude of the ultrasonic waves is from 1 µm to 100 µm both inclusive (a zero-to-peak value). The ultrasonic wave irradiation time is 1 minute or more, and may be preferably from 1 minute to 6 hours both inclusive.

The kind of electric waves is not particularly limited, but microwaves may be preferably used. This makes it possible to easily peel the layered substance from the layered laminate. In a case in which the microwaves are used, any microwave oven may be used, for example. Conditions of the microwaves such as output, frequency, and irradiation time are not particularly limited. To give an example, the output of the microwaves is 500 W, the frequency of the microwaves is 2.4 GHz, and the irradiation time is 10 seconds or more, and may be preferably from 10 seconds to 10 minutes both inclusive. Alternatively, low-energy microwaves may be used under conditions that the output is from 1 W to 100 W both inclusive, the frequency is 2.4 GHz, and the irradiation time is from 0.2 hours to 48 hours both inclusive.

One or more layered substances are peeled from the layered laminate by this irradiation process, and the layered substance is dispersed in the ionic liquid, thereby obtaining the layered substance-containing liquid. The layered laminate may or may not remain in the layered substance-containing liquid after being subjected to the irradiation process.

Note that in the irradiation process, changing the foregoing irradiation conditions (such as frequency) makes it possible to control the amount of peeling of the layered substance, that is, the concentration of the layered substance-containing liquid. Accordingly, setting the irradiation conditions to increase the amount of peeling of the layered substance makes it possible to obtain a high-concentration layered substance-containing liquid. More specifically, as the irradiation time increases, the amount of peeling of the layered substance increases, thereby increasing the concentration of the layered substance-containing liquid. Accordingly, the concentration of the layered substance-containing liquid is 10 mg/cm³ (=10 mg/ml) or more at the maximum, and may be preferably 20 mg/cm³ (=20 mg/ml) or more, and more preferably 40 mg/cm³ (=40 mg/ml) or more.

### <2-2. Purification of Layered Substance-containing Liquid>

The layered substance-containing liquid may be purified after preparation of the layered substance-containing liquid.

The layered substance-containing liquid may be purified, for example, by separating the layered substance-containing liquid (the ionic liquid after being subjected to the irradiation process) by centrifugation. However, the layered substance-containing liquid may be separated by centrifugation for a purpose other than purification. In this case, for example, any centrifugal separator may be used. It is possible to set any conditions for centrifugal separation. The layered substance-containing liquid may be separated by the centrifugal separation process into, for example, a solid phase containing the remaining layered laminate, impurities, and other material, and a liquid phase (a supernatant liquid) containing the layered substance. Note that in a case in which the layered substance-containing liquid is subjected to centrifugal separation, part or the entirety of the layered substance-containing liquid may be subjected to centrifugal separation.

After the centrifugal separation process, the liquid phase may be collected from the layered substance-containing liquid. Thus, the impurities and other materials are removed from the layered substance-containing liquid to purify the layered substance-containing liquid. In this case, changing the centrifugal separation conditions makes it possible to adjust the concentration of the layered substance-containing liquid (purity of the layered substance).

### <3. Action and Effects>

According to the foregoing layered substance-containing liquid and the foregoing producing method, the layered laminate is contained in the ionic liquid containing the cation represented by the formula (1), and the ionic liquid containing the layered laminate is irradiated with sonic waves or/and any other waves. In this case, irrespective of using simple processes such as a containing process and an irradiation process, the layered substance is easily peeled from the layered laminate; therefore, the layered substance is dispersed in the ionic liquid with a high concentration. Moreover, the layered substance is peeled stably and reproducibly. Accordingly, the number of layers of the layered substance is equalized, and the layered substance is resistant to breakage during peeling, which results in a sufficiently large area of the layered substance. Thus, even in a case in which the layered substance contains two or more kinds of elements as constituent elements, it is possible to easily obtain a high-quality layered substance.

In particular, when, in the formula (1), the monovalent hydrocarbon group is a straight-chain alkyl group having 1 to 7 carbons, the divalent hydrocarbon group is a straight-chain alkylene group having 1 to 4 carbons, and each of n, ml, m2, and m3 is an integer of 30 or less, it is possible to easily obtain the ionic liquid.

Moreover, when the ionic liquid contains a hexafluorophosphate ion or any other ion as an anion, dispersibility and other properties of the layered substance are improved. This makes it possible to achieve a higher effect.

Further, the layered substance is easily peeled from the layered laminate with use of ultrasonic waves as sonic waves or/and microwaves as electric waves, which makes it possible to achieve a higher effect.

Furthermore, when the ionic liquid after being subjected to irradiation is subjected to centrifugal separation, and the liquid phase (the supernatant liquid) is collected from the ionic liquid after being subjected to the centrifugal separation, the purity of the layered substance is improved, which makes it possible to achieve a higher effect.

### [Examples]

Examples of the present invention are described in detail below in the following order; however, the present invention is not limited thereto.
1. Synthesis of Ionic Liquid
2. Production of Layered Substance-containing Liquid
3. Evaluation of Layered Substance-containing Liquid

### <1. Synthesis of Ionic Liquid>

### [Synthesis Example 1]

A compound 2 was synthesized as the ionic liquid by the following procedure.

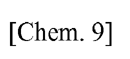

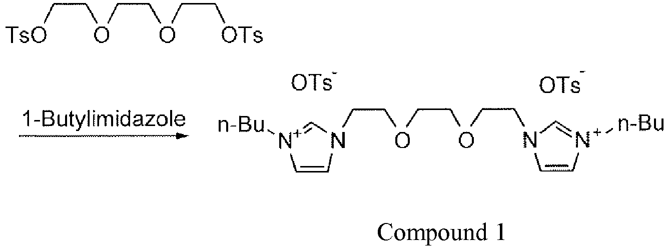

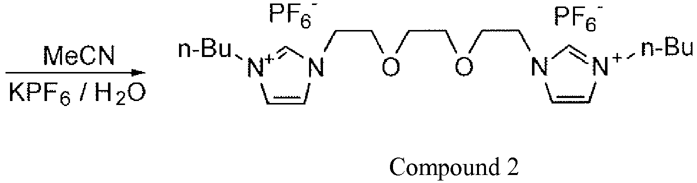

First, a compound 1 used to synthesize the compound 2 was synthesized. In this case, after 1-butylimidazole (37.159 g, 0.299 mol) was mixed into an acetonitrile solution (40 ml) of triethylene glycol bis(p-toluenesulfonate ester) (62.369 g, 0.136 mol) in an atmosphere of argon gas (Ar), a resultant mixture was stirred (at 60°C for 72 hours). Thereafter, the mixture (reactant) was concentrated and dried in a reduced pressure environment, and then methylene chloride (10 ml) was added to the mixture (viscous residue). Thereafter, the mixture was subjected to two-layer separation using ethyl acetate (50 ml) three times to obtain an ionic liquid phase. Subsequently, the ionic liquid phase was dried overnight in a vacuum oven (105°C) using phosphorus pentoxide (P₂O₅) together with a rotary evaporator. Thus, the compound 1 was obtained. Note that "Ts" and "n-Bu" in Chem. 7 respectively indicate a p-toluenesulfonate group and an n-butyl group.

The compound 1 was a pale yellow viscous liquid, and a yield of the compound 1 was 81% (579 g, 0.129 mol). Note that when the compound 1 was analyzed by a nuclear magnetic resonance (NMR) method, the following result was obtained.
¹H-NMR (500 MHz, DMSO-d6, 25°C) d (ppm) 9.7 (s, 2H), 7.81 (t, J = 1.5 Hz, 2H), 7.74 (t, J = 1.5 Hz, 2H), 7.48 (d, J = 8.5 Hz, 4H), 7.2 (d, J = 7.9 Hz, 4H), 4.33 (t, J = 4.9 Hz, 4H), 4.17 (t, J = 7.0 Hz, 4H), 3.73 (t, J = 4.9 Hz, 4H), 3.51 (s, 4H), 2.29 (s, 6H), 1.75 (dt, J = 15.0, 7.0 Hz, 4H), 1.22 (td, J = 15.0, 75 Hz, 4H), 0.88 (t, J = 7.3 Hz, 6H)
¹³C-NMR (125 MHz, DMSO-d6, 25°C) d (ppm) 145.7, 137.6, 136.3, 125.5, 122.8, 122.3, 69.3, 68.1, 48.7, 48.5, 31.3, 20.8, 18.7, 13.2
ESI-MS: m/z535.29 ([M-OTs]⁺, calcd. for C₂₇H₄₃N₄O₅S⁺ 535.30)

Next, the compound 2 was synthesized using the above-described compound 1. In this case, after an aqueous solution of potassium hexafluorophosphate (KPF₆/H₂O, 239 g, 0.125 mol) was mixed into an acetonitrile solution (MeCN, 30 ml) of the compound 1 (38.179 g, 0.054 mol), a resultant mixture was stirred (at room temperature for 2 hours). Thereafter, the mixture was left standing to separate the mixture (reactant) into a water phase and an ionic liquid phase. Subsequently, the supernatant water phase was removed from the mixture. Thereafter, methylene chloride (10 ml) was added to the ionic liquid phase, and then the ionic liquid phase was cleaned using distilled water (30 ml) three times. Then, after the ionic liquid phase was dried with use of sodium sulfate (Na₂SO₄), the ionic liquid phase was dried overnight in a vacuum oven (105°C) using phosphorus pentoxide together with a rotary evaporator. Thus, the compound 2 was obtained.

The compound 2 was a viscous liquid, and a yield of the compound 2 was 85% (30.39 g, 0.046 mol). Note that when the compound 2 was analyzed by the NMR method, the following results were obtained.
¹H-NMR (500 MHz, DMSO-d6, 25°C) d (ppm) 9.3 (s, 2H), 7.79 (t, J = 1.8 Hz, 2H), 7.72 (t, J = 1.8 Hz, 2H), 4.33 (t, J = 4.9 Hz, 4H), 4.8 (t, J = 7.0 Hz, 4H), 3.74 (t, J = 5.2 Hz, 4H), 3.52 (s, 4H), 1.77 (dt, J = 15.3, 6.9 Hz, 4H), 1.25 (td, J = 15.0, 7.3 Hz, 4H), 0.90 (t, J = 7.3 Hz, 6H)
¹³C-NMR (125 MHz, DMSO-d6, 25°C) d (ppm) 136.3, 122.8, 122.2, 69.3, 68.1, 48.8, 48.6, 31.3, 18.7, 13.2
ESI-MS: m/z509.25 ([M-PF₆]⁺, calcd. for C₂₀H₃₆F₆N₄O₂P⁺ 509.25)

### [Synthesis Examples 2 to 4]

Moreover, compounds 3 to 5 were synthesized as ionic liquids by a procedure described in International Publication No. WO 2014/175449.

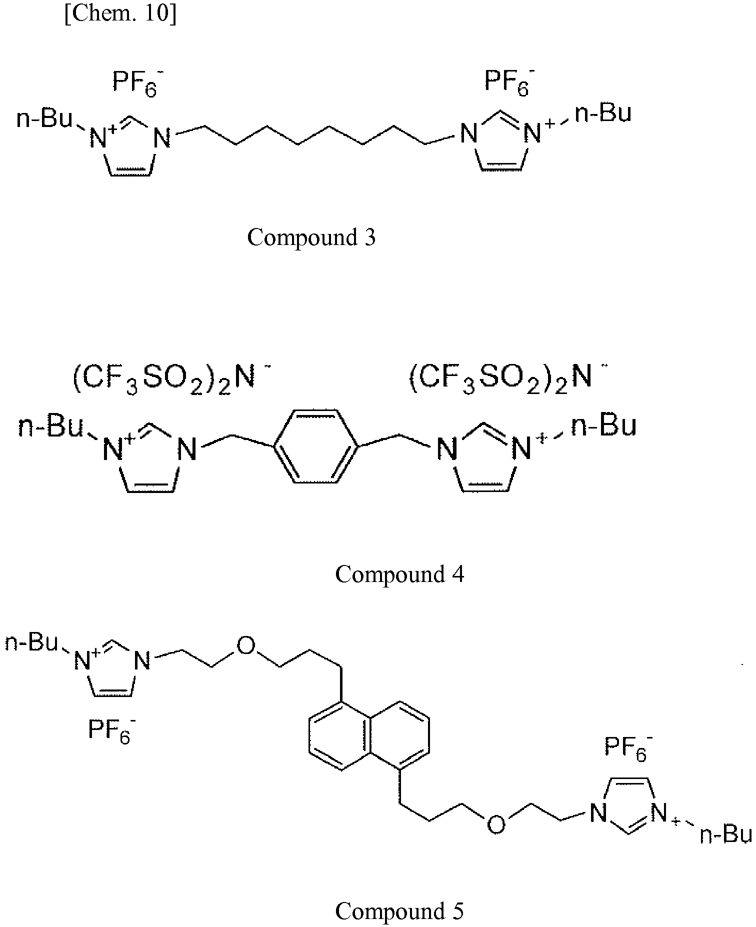

### <2. Production of Layered Substance-containing Liquid>

Next, the layered substance-containing liquids were manufactured using the above-described ionic liquids (compounds 2 to 5).

### (Experimental Examples 1 to 4)

First, 13 mg of a layered laminate (molybdenum disulfide: MoS₂) was mixed into 0.7 ml of the ionic liquid. The kind of the ionic liquid is as illustrated in Table 1. Subsequently, a resultant mixture was grated with use of a mortar (for 15 minutes) to obtain a mixture liquid in which the layered laminate was dispersed in the ionic liquid. In this case, molybdenum disulfide (an average particle diameter (D50) < 2 µm, purity = 99%) manufactured by Aldrich Japan Inc. was used. Subsequently, 0.58 g of the mixture liquid was put into a vial (0.5 ml) for a microwave synthesizer (Initiator+ manufactured by Biotage Japan Ltd.), and then, the vial was sealed. Thereafter, the mixture liquid was irradiated with microwaves with use of the microwave synthesizer. In this case, output was 17 W, and irradiation time was 6 hours. Thus, the layered substance-containing liquid was obtained.

### (Experimental Examples 5 to 12)

First, 25 mg of the layered laminate was mixed into 1 ml of the ionic liquid (the compound 2). The kind of the layered laminate is as illustrated in Table 1. More specifically, as the layered laminate, boron nitride (BN, manufactured by Aldrich Japan Inc., an average particle diameter D50=1 µm, purity=98%), tin disulfide (SnS₂, manufactured by Mitsuwa Chemicals Co., Ltd.), molybdenum telluride (MoTe₂, manufactured by Mitsuwa Chemicals Co., Ltd.), germanium sulfide (GeS, manufactured by Mitsuwa Chemicals Co., Ltd.), zirconium disulfide (ZrS₂, Mitsuwa Chemicals Co., Ltd.), niobium selenide (NbSe₂, manufactured by Mitsuwa Chemicals Co., Ltd.), talc (manufactured by Wako Pure Chemical Industries, Ltd.), and synthetic mica (manufactured by Wako Pure Chemical Industries, Ltd.) were used. Thereafter, the layered substance-containing liquid was obtained through a procedure similar to that in Experimental Examples 1 to 4.

### <3. Evaluation of Layered Substance-containing Liquid>

Next, the layered substance-containing liquid was analyzed with use of an X-ray diffraction (XRD) method. In this case, each of the layered substance-containing liquid before being subjected to an irradiation process and the layered substance-containing liquid after being subjected to the irradiation process was applied to a surface of a sample plate to prepare a sample for analysis. Moreover, as the XRD method, a concentration method was adopted.

In an analysis result (an XRD chart) of the XRD method, for each kind of the layered laminate, a peak caused by presence of the layered laminate was detected near a specific 2θ value. When a decrease rate (%) of intensity of a peak after the irradiation process was examined on the basis of the XRD chart, results illustrated in Table 1 were obtained. In order to examine the decrease rate, an intensity of a peak before the irradiation process and an intensity of a peak after the irradiation process were measured, and thereafter, the degrease rate was calculated on the basis of the intensities. Note that "2θ (°)" in Table 1 indicates a position of the above-described peak (a diffraction angle 2θ).

**[Table 1]**

| Experimental Example | Ionic Liquid | Layered Laminate | 2θ (°) | Decrease Rate (%) |
|---|---|---|---|---|
| 1 | Compound 2 | MoS₂ | 14.3 | 78.9 |
| 2 | Compound 3 | MoS₂ | 14.3 | 14.6 |
| 3 | Compound 4 | MoS₂ | 14.3 | 43.5 |
| 4 | Compound 5 | MoS₂ | 14.3 | 70.2 |
| 5 | Compound 2 | BN | 26.7 | 69.5 |
| 6 | Compound 2 | SnS₂ | 15 | 50.4 |
| 7 | Compound 2 | MoTe₂ | 12.6 | 45.8 |
| 8 | Compound 2 | GeS | 34.1 | 81.3 |
| 9 | Compound 2 | ZrS₂ | 15.1 | 94.2 |
| 10 | Compound 2 | NbSe₂ | 14 | 47.5 |
| 11 | Compound 2 | Talc | 9.4 | 36.1 |
| 12 | Compound 2 | Synthetic Mica | 6.24 | 73.1 |

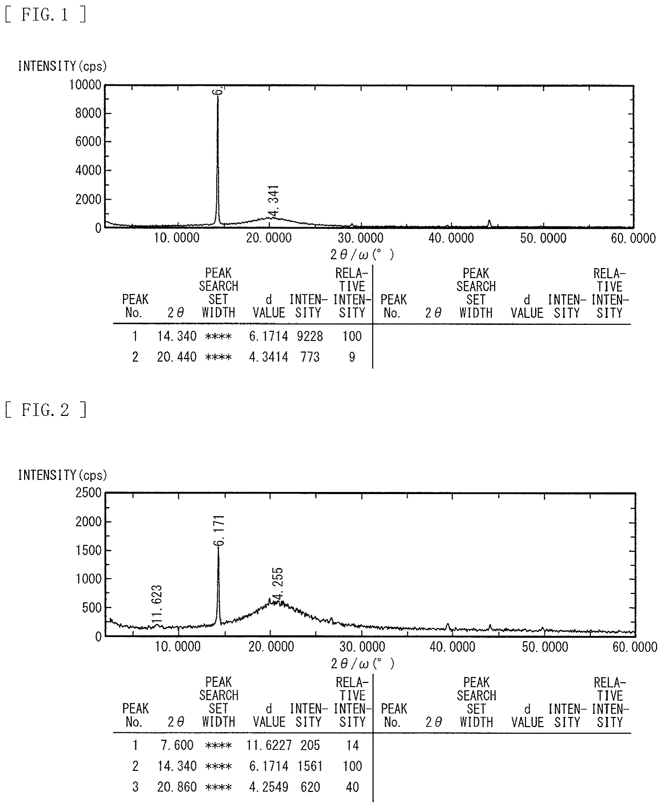

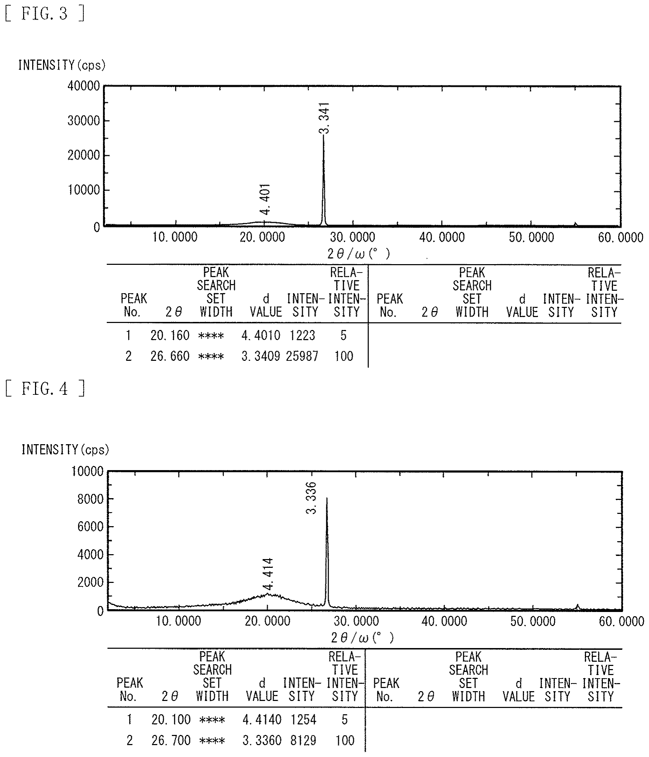

Note that, for reference, FIGs. 1 to 4 each illustrate an example of the XRD chart. FIG. 1 illustrates an XRD chart of Experimental Example 1 (MoS₂, before irradiation with microwaves, and FIG. 2 illustrates an XRD chart of Experimental example 1 (MoS₂, after irradiation with microwaves). FIG. 3 illustrates an XRD chart of Experimental Example 2 (BN, before irradiation with microwaves), and FIG. 4 illustrates an XRD chart of Experimental Example 2 (BN, after irradiation with microwaves).

In a case in which MoS₂ was used as the layered laminate (FIGs. 1 and 2), a peak caused by presence of the layered laminate was detected near 2θ = about 14.3°. Moreover, in a case in which BN was used as the layered laminate (FIGs. 3 and 4), a peak caused by presence of the layered laminate was detected near 2θ = about 26.7°.

As can be seen from the results illustrated in Table 1, a decrease rate of about 6% or more was obtained independent of the kind of the layered laminate. In other words, the intensity of the peak after the irradiation process was decreased, as compared with the intensity of the peak after the irradiation process. This indicates that the layered substance was peeled from the layered laminate to decrease the number of layered laminates and the number of layers of each of the layered laminates.

From these results, when the layered laminate is dispersed in an ionic liquid containing a specific cation, and the ionic liquid containing the layered laminate is irradiated with sonic waves or/and any other waves, the layered substance is easily obtained even in a case in which the layered substance contains two or more kinds of elements as constituent elements.

Although description of the present invention has been made by giving the embodiments and the examples as mentioned above, the present invention is not limited thereto and may be modified in a variety of ways.

This application claims the priority on the basis of Japanese Patent Application No. 2014-085213 filed on April 17, 2014 with Japan Patent Office, the entire contents of which are incorporated in this application by reference.

It should be understood by those skilled in the art that various modifications, combinations, sub-combinations, and alterations may occur depending on design requirements and other factors insofar as they are within the scope of the appended claims or the equivalents thereof.

## Claims

1. A layered substance-containing liquid comprising:
an ionic liquid containing a cation represented by the following formula (1); and
a layered substance containing two or more kinds of elements as constituent elements, 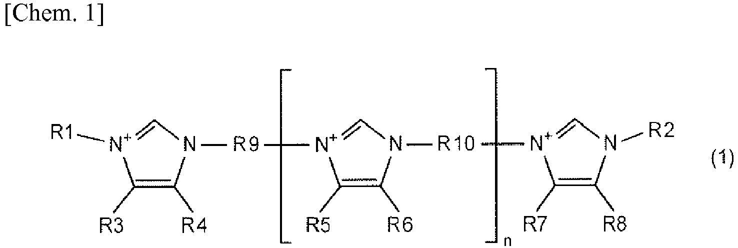 where each of R1 and R2 is a monovalent hydrocarbon group, each of R3 to R8 is one of a hydrogen atom and a monovalent hydrocarbon group, each of R9 and R10 is one of a divalent group represented by the following formula (2) and a divalent group represented by the following formula (3), and n is an integer of 0 or more, 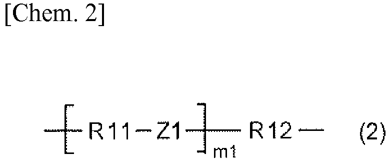 where each of R11 and R12 is a divalent hydrocarbon group, Z1 is one of an ether bond, a thioether bond, a divalent aromatic hydrocarbon group, and m1 is an integer of 0 or more, 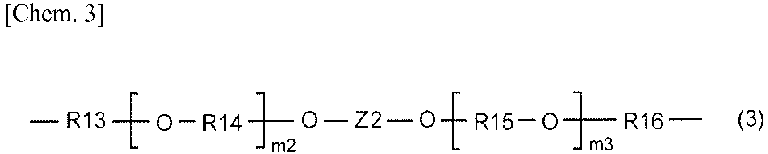 where each of R13 to R16 is a divalent hydrocarbon group, Z2 is a divalent aromatic hydrocarbon group, each of m2 and m3 is an integer of 1 or more.

2. A method for producing a layered substance-containing liquid comprising:
containing a laminate of layered substances in an ionic liquid containing a cation represented by the following formula (1), the layered substances each containing two or more kinds of elements as constituent elements; and
irradiating the ionic liquid containing the laminate with one or both of sonic waves and electric waves. 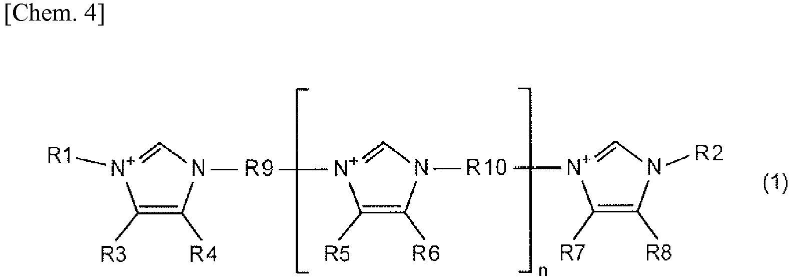 where each of R1 and R2 is a monovalent hydrocarbon group, each of R3 to R8 is one of a hydrogen atom and a monovalent hydrocarbon group, each of R9 and R10 is one of a divalent group represented by the following formula (2) and a divalent group represented by the following formula (3), and n is an integer of 0 or more, 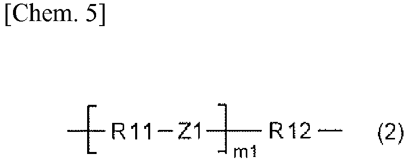 where each of R11 and R12 is a divalent hydrocarbon group, Z1 is one of an ether bond, a thioether bond, a divalent aromatic hydrocarbon group, and m1 is an integer of 0 or more, 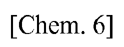 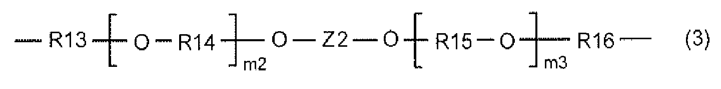 where each of R13 to R16 is a divalent hydrocarbon group, Z2 is a divalent aromatic hydrocarbon group, each of m2 and m3 is an integer of 1 or more.

3. The method for producing the layered substance-containing liquid according to claim 2, wherein ultorasonic waves are used as the sonic waves, and microwaves are used as the electric waves.

4. The method for producing the layered substance-containing liquid according to claim 2 or claim 3, further comprising subjecting the ionic liquid irradiated with one or both of the sonic waves and the electric waves to centrifugal separation.

5. The method for producing the layered substance-containing liquid according to claim 4, wherein a liquid phase is collected from the ionic liquid having been subjected to the centrifugal separation.
